# EUROPEAN PATENT APPLICATION

(11) **EP 4 831 280 A1**
(43) Date of publication of application: **28.10.2026**
(21) Application number: 24908364.3
(22) Date of filing: 18.12.2024
(51) Int. Cl.: C12N 5/077, A23L 13/00

(54) **CULTURE MEDIUM COMPOSITION FOR TRANSDIFFERENTIATION OF MUSCLE STEM CELLS INTO ADIPOCYTES, AND METHOD FOR PRODUCING CULTURED MEAT BY USING SAME**

(30) Priority: 18.12.2023 KR 20230184632
(71) Applicant: Hanwha Solutions Corporation, Jung-gu Seoul 04541 (KR)
(72) Inventor: KIM, Hyeong Taek, Seoul 04541 (KR); PARK, Jin Hee, Seoul 04541 (KR); LEE, Eunji, Seoul 04541 (KR); LEE, Seoha, Seoul 04541 (KR)
(74) Representative: Berggren Oy
(86) International application number: PCT/KR2024/097148
(87) International publication number: WO 2025/136060

(57) **Abstract**

The present invention relates to a culture medium composition for transdifferentiation of muscle stem cells into adipocytes, and a method for producing cultured meat by using same, and, more specifically, to: a culture medium composition for transdifferentiation of muscle stem cells into adipocytes, comprising a combination of two or more selected from the group consisting of oleic acid, thiazolidinedione, and lecithin; a method for transdifferentiation of muscle stem cells into adipocytes by using the medium composition; a method for producing cultured meat by using the transdifferentiation method; cultured meat produced by the cultured meat production method; and a food composition comprising same.

## Description

### TECHNICAL FIELD

This application claims priority to and the benefit of Korea Patent Application No. 10-2023-0184632, filed on December 18, 2023, the disclosure of which is incorporated herein by reference in its entirety.

The present invention relates to a culture medium composition for transdifferentiating muscle stem cells into adipocytes and a method for producing cultured meat using the same, and more specifically to a culture medium composition for transdifferentiating muscle stem cells into adipocytes, including a combination of two or more selected from the group consisting of oleic acid, thiazolidinedione and lecithin, a method for transdifferentiating muscle stem cells into adipocytes using the medium composition, a method for producing cultured meat using the transdifferentiating method, cultured meat produced by the method for producing cultured meat, and a food composition including the same.

### BACKGROUND ART

According to a recent report published by the Food and Agriculture Organization of the United Nations (FAO), the world's population is expected to increase by 0.6% annually from 7.64 billion in July 2018 to 9.2 billion in 2050, and in line with this population increase, human meat consumption is also estimated to reach 465 million tons.

Accordingly, the Food and Agriculture Organization of the United Nations (FAO) announced that additional 200 million tons of production is needed each year to meet the increasing demand for meat. In addition, recently, there have been many cases of livestock diseases such as mad cow disease and foot-and-mouth disease, and pathogens contained in livestock intestines not being killed during the processing process, thereby causing various diseases, which is causing major social problems.

Recently, 'cultured meat' has been gaining attention as a solution to the problems of population growth and disease outbreaks in slaughtered livestock.

Cultured meat refers to edible meat obtained by extracting cells from living animals and multiplying them using cell engineering technology. It can be said to be a branch of cellular agriculture that obtains meat without going through the process of raising livestock. Cultured meat is also called alternative meat or artificial meat, and is called *in vitro* meat because it is grown in a test tube, clean meat because it is produced in a clean production facility rather than a traditional breeding facility, and lab-grown meat because some prototypes are made in laboratories.

Cultured meat uses tissue engineering technology among cell engineering technologies. Cells are obtained from living animals, but they are obtained painlessly and stem cell culture is used. Therefore, cultured meat is an ideal food production technology that saves animals while also reducing environmental pollution.

The biggest problem in the process of producing cultured meat is that it cannot be exactly replicated in meat with actual intramuscular fat. As far as is known, it is necessary to culture muscle cells and fat cells separately and mix the resulting muscle and fat, and thus, it is impossible to replicate the texture of meat with intramuscular fat, and there is a problem that it can only be processed into minced meat.

Accordingly, there have been attempts to produce cultured meat that mimics meat with intramuscular fat by co-culturing muscle and fat, but most of them have been conducted by studying different cell-to-cell or intracellular interactions by culturing muscle cell lines such as C2C12 together with preadipocytes and adding factors necessary for culturing each fat and muscle cell (Non-Patent Document 1).

Under these circumstances, the inventors of the present invention have made extensive efforts to develop a medium composition capable of transdifferentiating muscle stem cells into adipocytes in order to produce cultured meat in which intramuscular fat is present simultaneously. As a result, it was confirmed that when a combination of two or more of oleic acid, thiazolidinedione and lecithin is treated in the medium composition, muscle stem cells are effectively transdifferentiated into adipocytes, thereby completing the present invention.

### [Related Art Documents]

### [Non-Patent Documents]

(Non-Patent Document 1) Anim Sci J. 2019 Mar;90(3):423-434.

### DISCLOSURE

### TECHNICAL PROBLEM

Accordingly, an object of the present invention is to provide a culture medium composition for transdifferentiating muscle stem cells into adipocytes, including two or more of oleic acid, thiazolidinedione and lecithin.

Another object of the present invention is to provide a method for transdifferentiating muscle stem cells into adipocytes using the above-described medium composition.

Still another object of the present invention is to provide a method for producing cultured meat using the method of transdifferentiating the above-described muscle stem cells into adipocytes, and cultured meat produced thereby.

Still another object of the present invention is to provide a food composition including the cultured meat described above.

### TECHNICAL SOLUTION

In order to solve the above-described problems, the present invention provides a culture medium composition for transdifferentiating muscle stem cells into adipocytes, including two or more selected from the group consisting of oleic acid, thiazolidinedione and lecithin.

In the present invention, the thiazolidinedione may be one or more selected from the group consisting of pioglitazone, rosiglitazone, lobeglitazone, troglitazone, ciglitazone, darglitazone, englitazone, netoglitazone and rivoglitazone.

In the present invention, the oleic acid may be included at a concentration of 100 to 250 µM.

In the present invention, the thiazolidinedione may be included at a concentration of 5 to 20 µM.

In the present invention, the lecithin may be included at a concentration of 5 to 35 µM.

In the present invention, when the culture medium composition includes oleic acid and thiazolidinedione, the oleic acid and thiazolidinedione may be included in a concentration ratio of 40 : 0.5 to 6.

In the present invention, when the culture medium composition includes oleic acid and lecithin, the oleic acid and lecithin may be included in a concentration ratio of 40 : 1 to 7.

In the present invention, when the culture medium composition includes oleic acid, thiazolidinedione and lecithin, the oleic acid, thiazolidinedione and lecithin may be included in a concentration ratio of 40 : 0.5 to 6 : 1 to 7.

In the present invention, the culture medium composition may further include one or more selected from the group consisting of insulin, 3-isobutyl-1-methylxanthine (IBMX) and dexamethasone.

In the present invention, the culture medium composition may not include serum or may further include serum.

In the present invention, when the culture medium composition does not include serum, the culture medium composition may further include one or more selected from the group consisting of insulin, transferrin and selenite.

In the present invention, the muscle stem cells may be passaged 1 to 25 times.

In addition, the present invention provides a method for transdifferentiating muscle stem cells into adipocytes using the culture medium composition described above.

Additionally, the present invention provides a method for producing cultured meat, using the method for transdifferentiating muscle stem cells into adipocytes described above and cultured meat produced thereby.

In the present invention, in the cultured meat, muscle cells and intramuscular fat may be present simultaneously.

Furthermore, the present invention provides a food composition, including the cultured meat described above.

### ADVANTAGEOUS EFFECTS

The culture medium composition for transdifferentiating muscle stem cells into adipocytes according to the present invention can effectively induce transdifferentiation from muscle stem cells into adipocytes by a simple combination of treating two or more of oleic acid, thiazolidinedione and lecithin, thereby inducing cells in which intramuscular fat is present simultaneously, and thus, it is possible to produce cultured meat with improved flavor.

### DESCRIPTION OF DRAWINGS

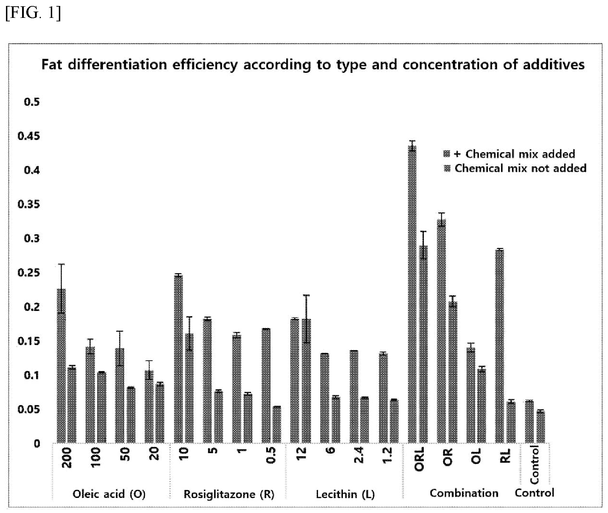

FIG. 1 shows the rate of adipocyte transdifferentiation according to the type and concentration of additives included in the medium (left graph: chemical mix added, right graph: chemical mix not added).

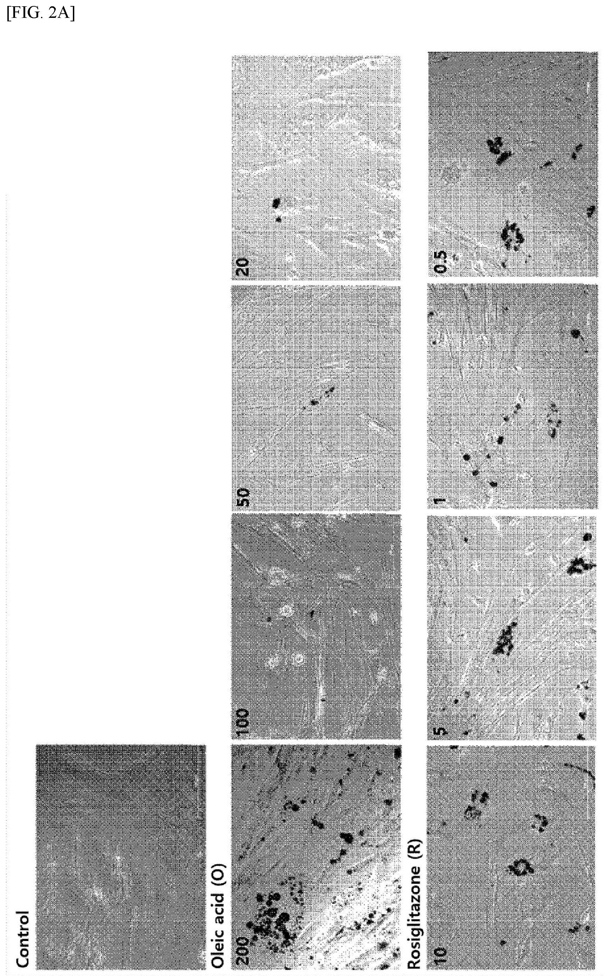

FIG. 2A shows the results of confirming the adipocyte transdifferentiation pattern by staining with Oil Red O when muscle stem cells were cultured in the following conditions: DMEM (Dulbecco's Modified Eagle's Medium High glucose) standard medium including FBS (Fetal Bovine Serum) treated with insulin, IBMX and dexamethasone (control group), the control medium additionally treated with oleic acid at various concentrations (respectively 20, 100, 50 and 200 µM), and the control medium additionally treated with rosiglitazone at various concentrations (respectively 0.5, 1, 5 and 10 µM).

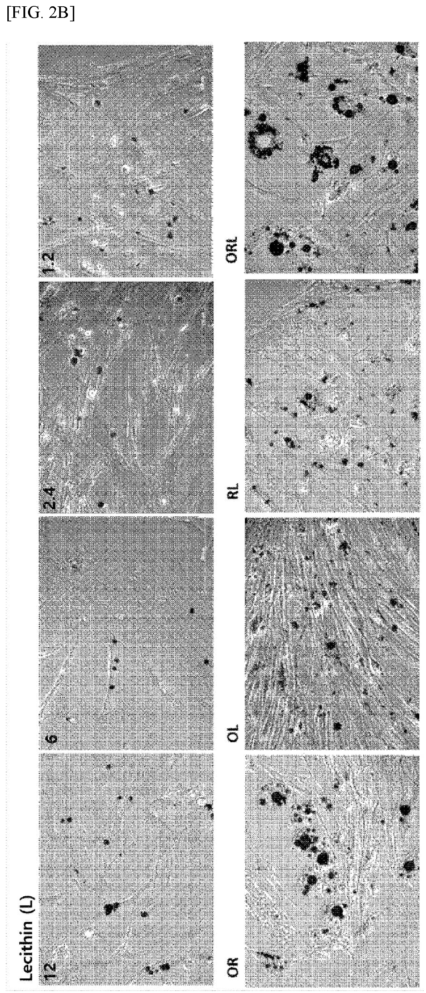

FIG. 2B shows the results of culturing muscle stem cells in the control medium additionally treated with lecithin at various concentrations (1.2, 2.4, 6 and 12 µg/mL, respectively) and in the control medium treated with combinations of oleic acid + rosiglitazone (OR), oleic acid + lecithin (OL), rosiglitazone + lecithin (RL) and oleic acid + rosiglitazone + lecithin (PRL), and confirming the transdifferentiation pattern into adipocytes by staining with Oil Red O.

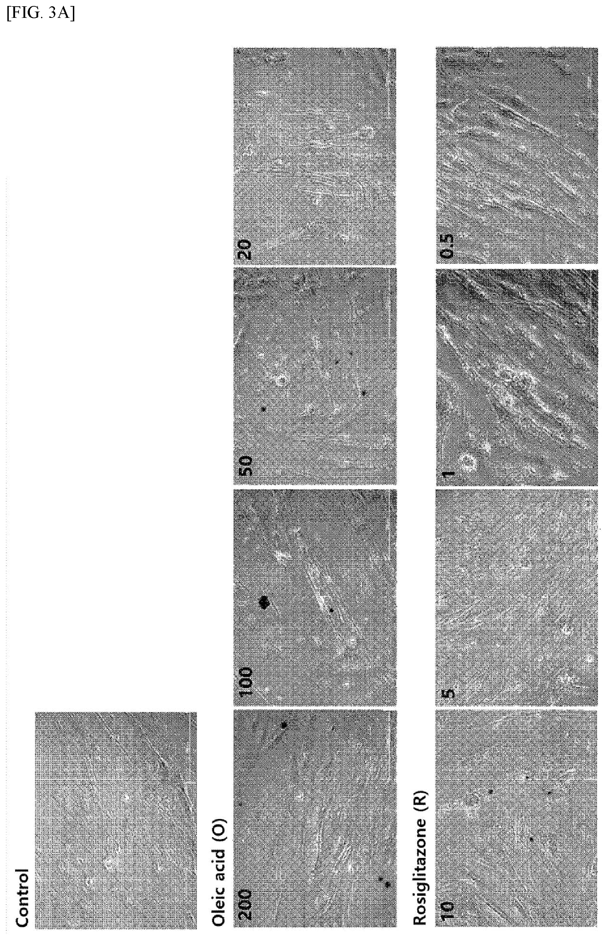

FIG. 3A shows the results of confirming the transdifferentiation pattern into adipocytes by staining with Oil Red O after culturing muscle stem cells in DMEM standard medium including FBS (control group), in the standard medium treated with oleic acid at various concentrations (20, 100, 50 and 200 µM, respectively), and in the standard medium treated with rosiglitazone at various concentrations (0.5, 1, 5 and 10 µM, respectively).

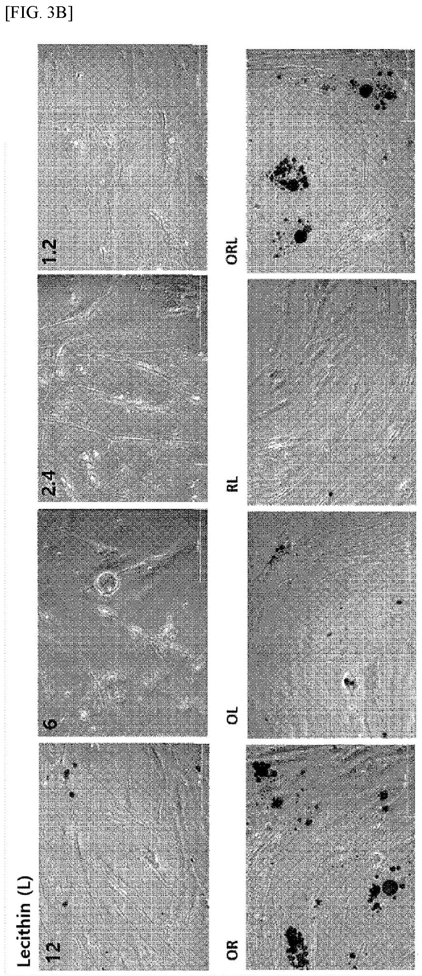

FIG. 3B shows the results of confirming the pattern of adipocyte transdifferentiation by staining with Oil Red O after culturing muscle stem cells in the standard medium treated with lecithin at different concentrations (1.2, 2.4, 6, and 12 µg/ml, respectively) and in the standard medium treated with combinations of oleic acid + rosiglitazone (OR), oleic acid + lecithin (OL), rosiglitazone + lecithin (RL) and oleic acid + rosiglitazone + lecithin (ORL), respectively.

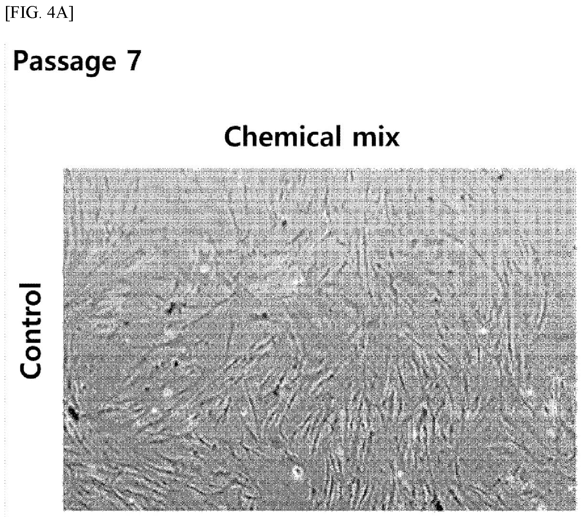

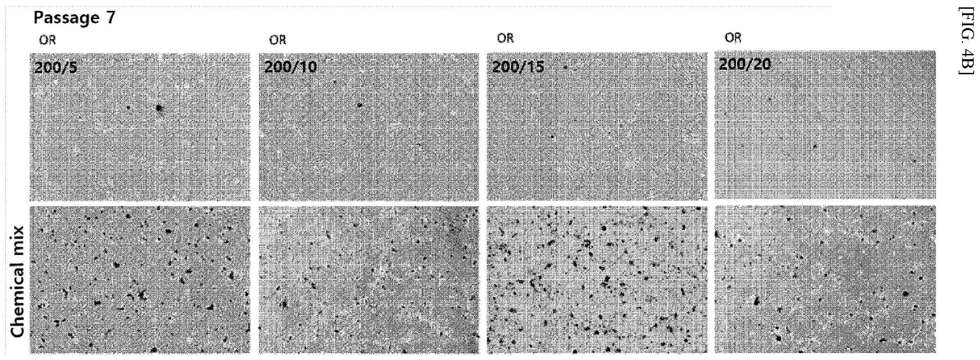

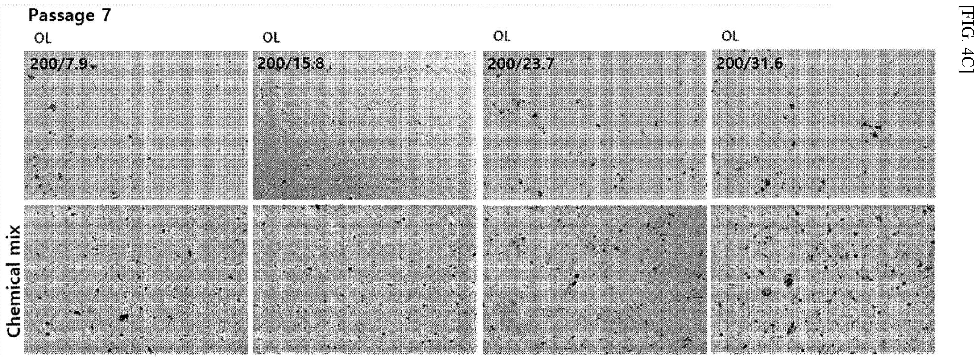

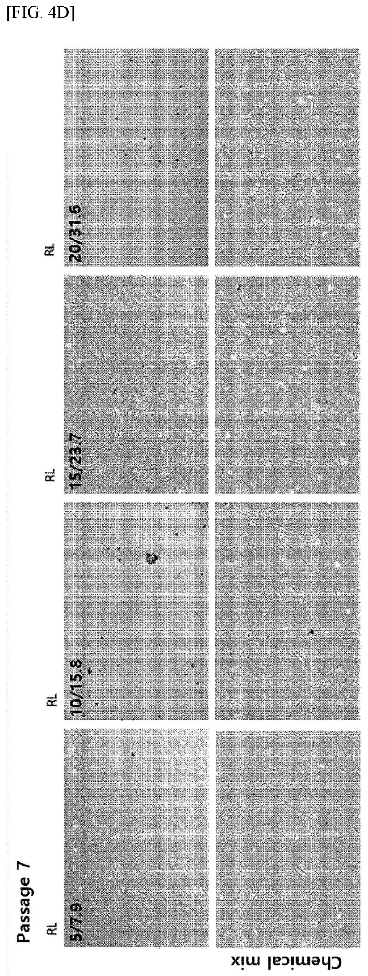

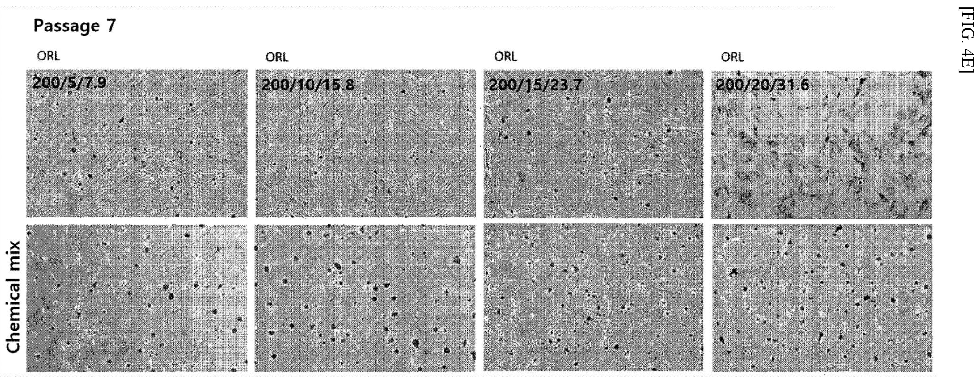

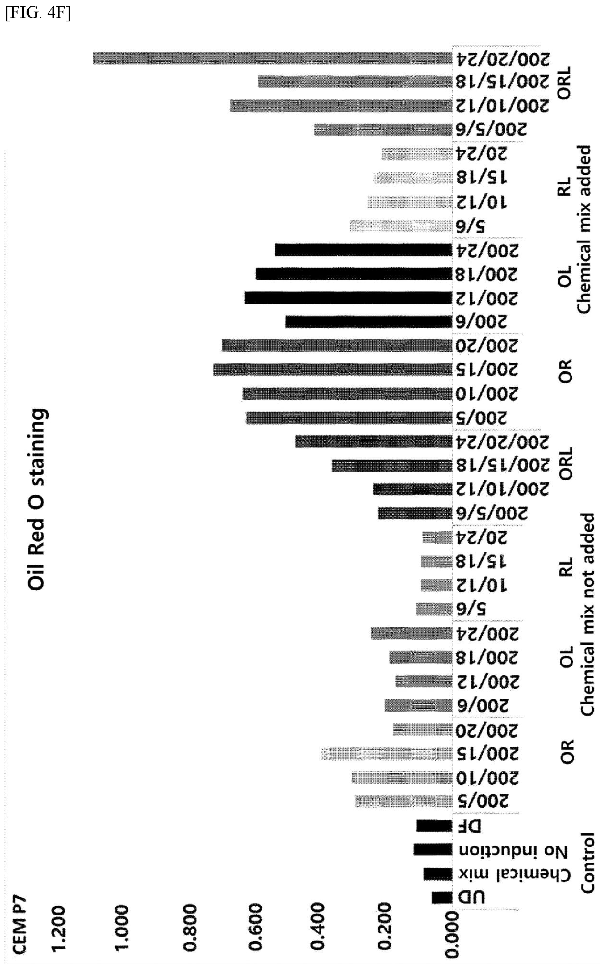

FIGS. 4A to 4F show the results of confirming the transdifferentiation pattern of muscle stem cells at passage 7 into adipocytes by staining with Oil Red O, FIG. 4A shows the result in a state (control group) treated with insulin, IBMX and dexamethasone (chemical mix) in the DMEM standard medium including FBS, FIG. 4B shows the result in a state treated with Combination 1 (oleic acid + rosiglitazone (OR): 200 µM + 5 / 10 / 15 / 20 µM) in the presence or absence of insulin, IBMX and dexamethasone in the DMEM standard medium including FBS, FIG. 4C shows the result in a state treated with Combination 2 (oleic acid + lecithin (OL): 200 µM + 7.9 / 15.8 / 23.7 / 31.6 µM) in the presence or absence of insulin, IBMX and dexamethasone in the DMEM standard medium including FBS, FIG. 4D shows the results in a state treated with Combination 3 (rosiglitazone + lecithin (RL) 5 / 10 / 15 / 20 µM + 7.9 / 15.8 / 23.7 / 31.6 µM) in the presence or absence of insulin, IBMX and dexamethasone in DMEM standard medium including FBS, FIG. 4E shows the results in a state treated with Combination 4 (oleic acid + rosiglitazone + lecithin (ORL): 200 µM + 5 / 10 / 15 / 20 µM + 7.9 / 15.8 / 23.7 / 31.6 µM) in the presence or absence of insulin, IBMX and dexamethasone in the DMEM standard medium including FBS, and FIG. 4F is a graph quantitatively showing the results of Oil Red O staining of each group.

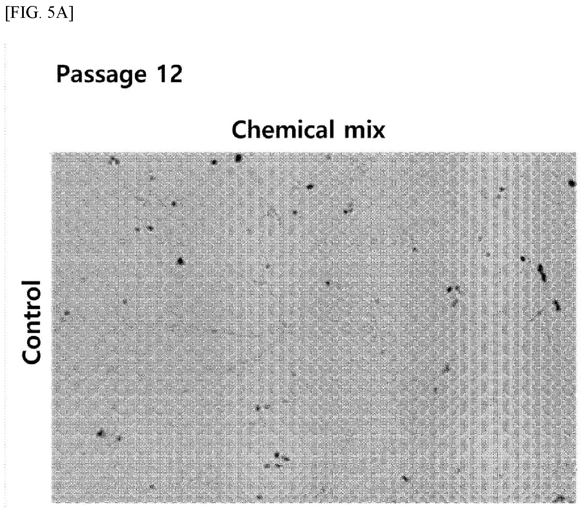

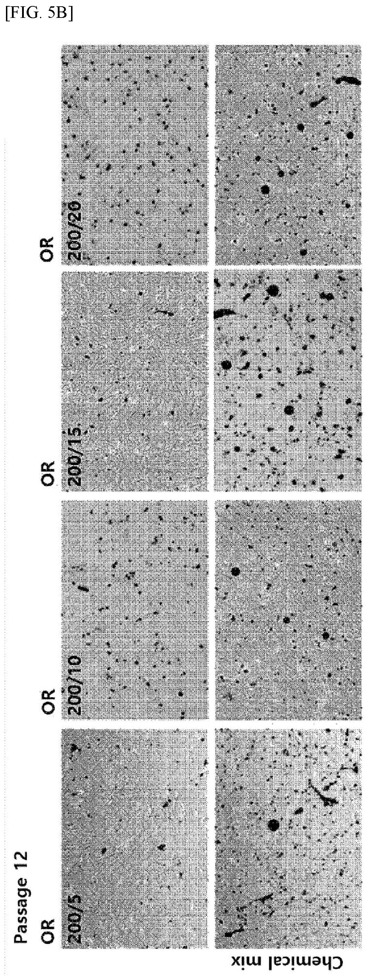

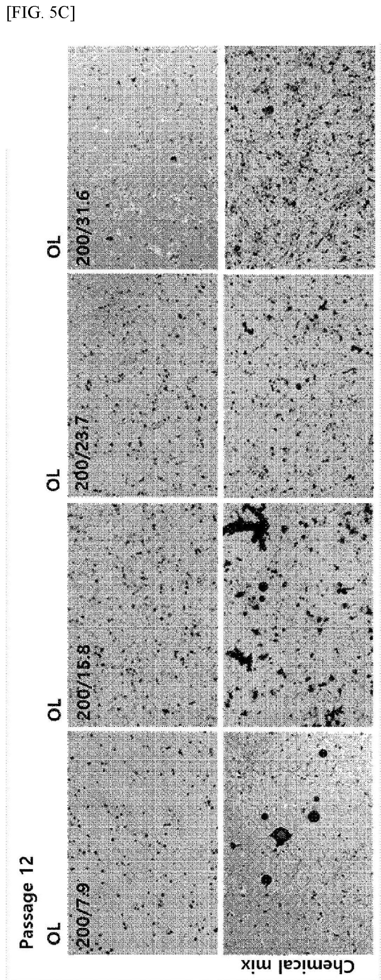

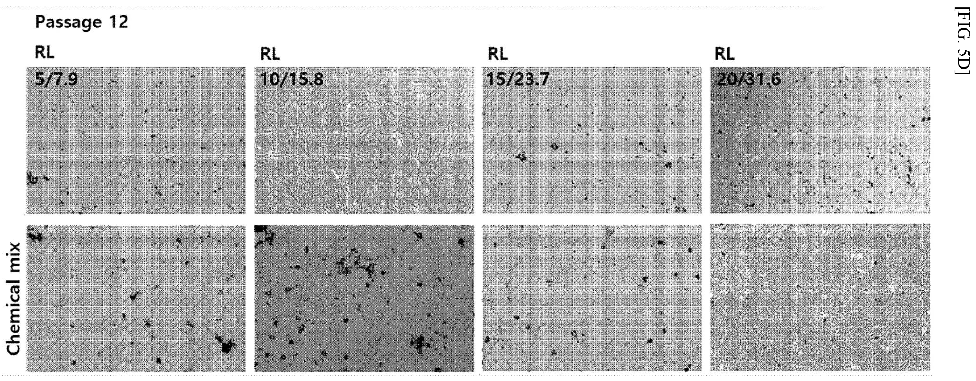

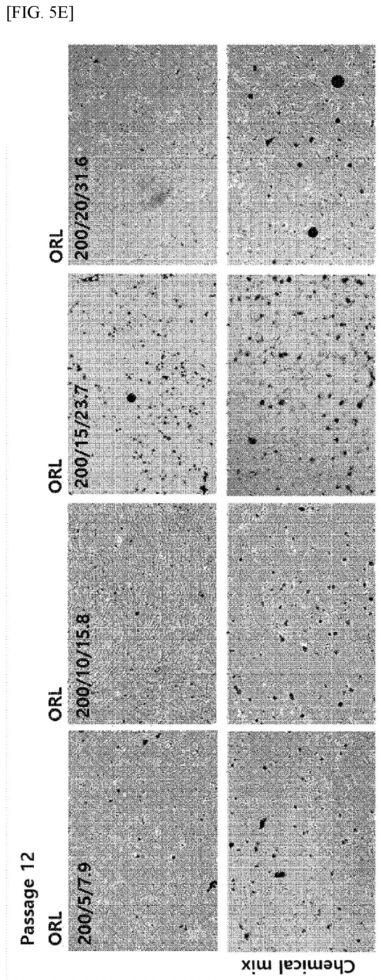

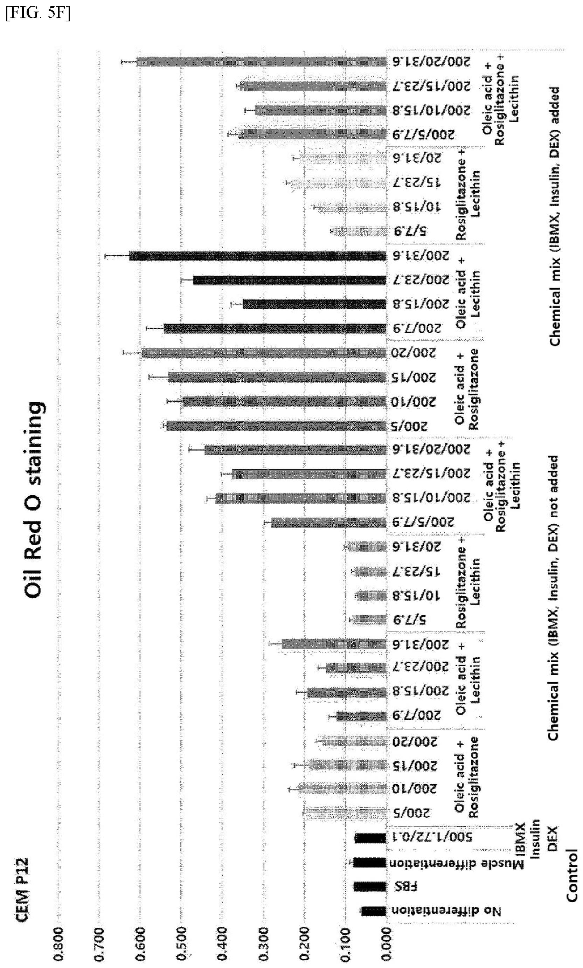

FIGS. 5A to 5F show the results of confirming the transdifferentiation pattern of muscle stem cells at passage 12 into adipocytes by staining with Oil Red O, FIG. 5A shows the result in a state (control group) treated with insulin, IBMX and dexamethasone (Chemical mix) in the DMEM standard medium including FBS, FIG. 5B shows the result in a state treated with Combination 1 (oleic acid + rosiglitazone (OR): 200 µM + 5 / 10 / 15 / 20 µM) in the presence or absence of insulin, IBMX and dexamethasone in the DMEM standard medium including FBS, FIG. 5C shows the result in a state treated with Combination 2 (oleic acid + lecithin (OL): 200 µM + 7.9 / 15.8 / 23.7 / 31.6 µM) in the presence or absence of insulin, IBMX and dexamethasone in the DMEM standard medium including FBS, FIG. 5D shows the results in a state treated with Combination 3 (rosiglitazone + lecithin (RL): 5 / 10 / 15 / 20 µM + 7.9 / 15.8 / 23.7 / 31.6 µM) in the presence or absence of insulin, IBMX and dexamethasone in DMEM standard medium including FBS, FIG. 5E shows the results in a state treated with Combination 4 (oleic acid + rosiglitazone + lecithin (ORL): 200 µM + 5 / 10 / 15 / 20 µM + 7.9 / 15.8 / 23.7 / 31.6 µM) in the presence or absence of insulin, IBMX and dexamethasone in the DMEM standard medium including FBS, and FIG. 5F is a graph quantitatively showing the results of Oil Red O staining of each group.

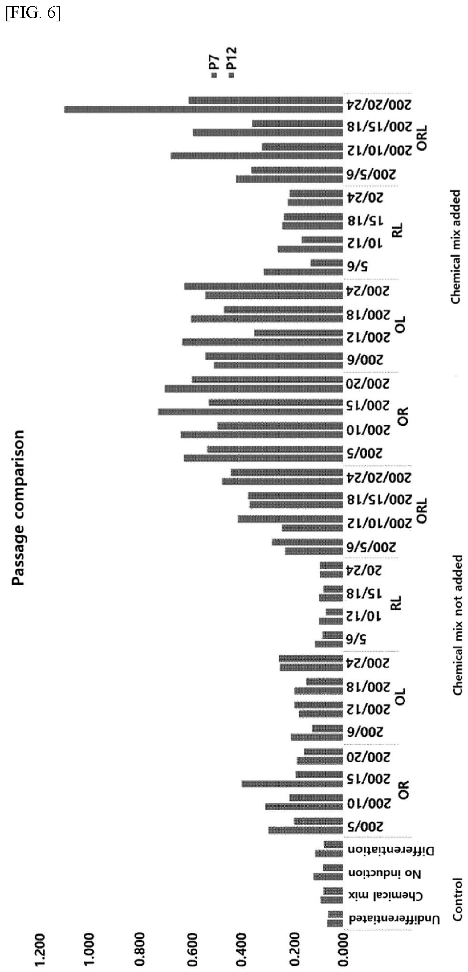

FIG. 6 is a graph comparing the results of FIGS. 4F and 5F according to the passage number (left graph: P7, right graph: P12).

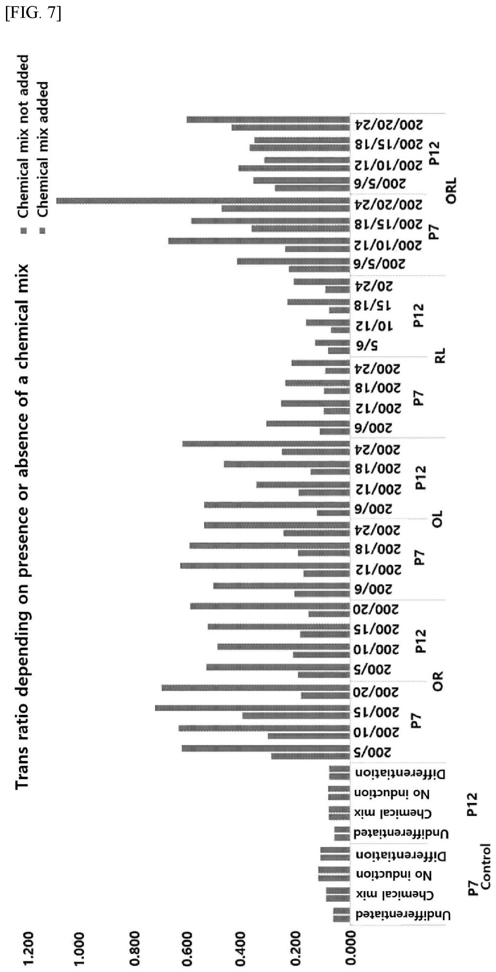

FIG. 7 is a graph comparing the results of FIGS. 4F and 5F according to the presence or absence of chemical mix (insulin, IBMX and dexamethasone) (left graph: chemical mix added, right graph: chemical mix not added).

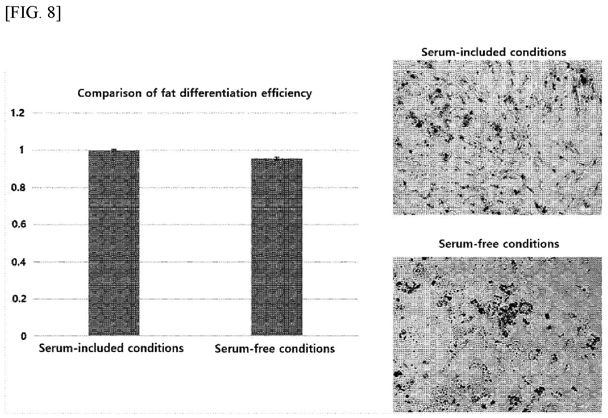

FIG. 8 shows the results of staining with Oil Red O to determine the rate of transdifferentiation into adipocytes and the differentiation pattern of muscle stem cells depending on the presence or absence of serum in the medium.

### BEST MODE

Hereinafter, the present invention will be described in more detail.

All technical terms used in the present invention, unless otherwise defined, are used with the same meanings as commonly understood by those skilled in the art in the relevant field of the present invention. In addition, although preferred methods or samples are described in the present specification, similar or equivalent ones are also included in the scope of the present invention.

The present invention relates to a culture medium composition for transdifferentiating muscle stem cells into adipocytes, including two or more selected from the group consisting of oleic acid, thiazolidinedione and lecithin.

In addition, the present invention relates to a culture medium composition including two or more selected from the group consisting of oleic acid, thiazolidinedione and lecithin for use in transdifferentiating muscle stem cells into adipocytes.

Additionally, the present invention relates to the use of a composition including two or more selected from the group consisting of oleic acid, thiazolidinedione and lecithin in the preparation of a culture medium for transdifferentiating muscle stem cells into adipocytes.

It should be understood that although certain aspects are described herein with the term "including", other similar aspects described in terms of "consisting of" and/or "consisting essentially of" are also provided.

In the present invention, the terms "medium", "culture medium", "culture medium", "medium composition", "culture composition" and "culture medium composition" mean a culture solution including nutrients capable of supporting the growth and survival of stem cells under *in vitro* culture conditions, and are not distinguished in the present specification and may be used interchangeably.

The term "muscle stem cells" in the present invention includes precursor cells such as quiescent satellite cells in a growth-quiescent state and activated satellite cells called myoblasts. Muscle stem cells may differentiate into muscle cells, and in particular, muscle stem cells derived from livestock may be used for actual cultured meat production and the like, and are cells with very high utility value in agricultural biology.

In the present invention, the term "culturing of muscle stem cells" means cell proliferation of muscle stem cells while maintaining their differentiation potential, which is stemness.

In the present invention, the thiazolidinedione may be selected from the group consisting of pioglitazone, rosiglitazone, lobeglitazone, troglitazone, ciglitazone, darglitazone, englitazone, netoglitazone and rivoglitazone.

In a specific embodiment of the present invention, in order to confirm the adipocyte transdifferentiation rate when oleic acid, rosiglitazone and lecithin were treated at different concentrations in the presence or absence of a combination of compounds (insulin, IBMX and dexamethasone) and when two or more thereof were treated in combination, Oil Red O staining was performed. As a result, as shown in FIGS. 1, 2A, 2B, 3A and 3B, the adipocyte transdifferentiation efficiency increased as the concentrations of oleic acid, rosiglitazone and lecithin increased regardless of the presence or absence of IBMX, insulin and dexamethasone, and in particular, when IBMX, insulin and dexamethasone were included, the adipocyte transdifferentiation efficiency increased significantly. It was confirmed that the combination showing the best adipocyte transdifferentiation rate was the case including all of IBMX, insulin, dexamethasone, oleic acid, rosiglitazone and lecithin.

Accordingly, the medium composition according to the present invention may include two or more selected from the group consisting of oleic acid, rosiglitazone and lecithin, in the presence or absence of IBMX, insulin and dexamethasone.

In another specific embodiment of the present invention, in order to confirm the efficiency of adipocyte transdifferentiation according to the concentration ratio of the combination of substances added to the medium in the presence or absence of a combination of compounds (insulin, IBMX and dexamethasone), passage 7 muscle stem cells were cultured in a medium treated with a combination of substances at various concentration ratios, and then, Oil red O staining was performed to evaluate the adipocyte transdifferentiation rate. As a result, as shown in FIGS. 4A to 4F, it was confirmed that the adipocyte transdifferentiation rate significantly increased when Combination 1 (oleic acid + rosiglitazone (OR): 200 µM + 5 / 10 / 15 / 20 µM), Combination 2 (oleic acid + lecithin (OL): 200 µM + 7.9 / 15.8 / 23.7 / 31.6 µM) or Combination 4 (oleic acid + rosiglitazone + lecithin (ORL): 200 µM + 5 / 10 / 15 / 20 µM + 7.9 / 15.8 / 23.7 / 31.6 µM) was treated in the standard medium including IBMX and dexamethasone.

Accordingly, the medium composition according to the present invention may include oleic acid at a concentration of 100 to 250 µM, preferably 150 to 250 µM, and most preferably 180 to 230 µM, but is not limited thereto. If oleic acid is included at a concentration of less than 100 µM, the adipocyte transdifferentiation rate may be significantly reduced, and if oleic acid is included at a concentration of more than 250 µM, the effect of increasing the adipocyte transdifferentiation rate according to the increase in concentration is minimal, and cytotoxicity may occur, resulting in the problem of muscle stem cells dying.

The medium composition according to the present invention may include thiazolidinedione at a concentration of 5 to 20 µM, and the optimal concentration may be adjusted within the above range depending on the presence of other additional components.

The medium composition according to the present invention may include lecithin at a concentration of 5 to 35 µM, and the optimal concentration may be adjusted within the above range depending on the presence of other additional components.

In the present invention, when the medium composition includes oleic acid and thiazolidinedione, the oleic acid and thiazolidinedione may be included in a concentration ratio of 40 : 0.5 to 6, preferably 40 : 1 to 4, and most preferably 40 : 2.5 to 4. When the concentration ratio is out of the above, the synergistic effect of the adipocyte transdifferentiation rate according to the combination may decrease.

In the present invention, when the medium composition includes oleic acid and lecithin, the oleic acid and lecithin may be included in a concentration ratio of 40 : 1 to 7, preferably 40 : 1.4 to 6.6, and most preferably 40 : 3 to 6.4. When the concentration ratio is out of the above range, the synergistic effect of the adipocyte transdifferentiation rate according to the combination may decrease.

In the present invention, when the medium composition includes oleic acid, thiazolidinedione and lecithin, the oleic acid, thiazolidinedione and lecithin may be included in a concentration ratio of 40 : 0.5 to 6 : 1 to 7, preferably 40 : 2 to 6 : 3 to 6.6, more preferably 40 : 2 to 4 : 3 to 6.6, and most preferably 40 : 2 to 4 : 3 to 6.4. When the concentration ratio is out of the above ranges, the synergistic effect of the adipocyte transdifferentiation rate according to the combination may be significantly reduced.

In the present invention, the medium composition may further include any one or more selected from the group consisting of insulin, 3-isobutyl-1-methylxanthine (IBMX) and dexamethasone.

In the present invention, the medium composition may not include serum, or may further include serum. In this case, the serum may be, for example, serum of various animals, and may be chicken serum, horse serum, fetal calf serum (FCS) or fetal bovine serum (FBS), but is not limited thereto. The content of the serum may be 5% to 20%, and preferably 5% to 15%, and here, % means volume (v/v)%.

In the present invention, when the medium composition does not include serum, it may further include one or more selected from the group consisting of insulin, transferrin and selenite, and most preferably, it may include all three of insulin, transferrin and selenite.

The muscle stem cells used in the medium composition according to the present invention may be muscle stem cells that have been passaged 1 to 25 times, but are not limited thereto.

The term "subculture" used in the present invention refers to a method of continuously culturing cells in a healthy state for a long period of time by periodically transferring a portion of cells to a new culture vessel and then changing the culture medium to continuously culture the cells. The term "passage" refers to growth from the initial seed culture in a culture vessel to the time when the cells grow vigorously (confluence) in the same culture vessel. As the number of cells increases in a culture vessel with a limited space, when a certain period of time passes, the growth nutrients are consumed or contaminants accumulate, causing the cells to naturally die, and thus, it is used as a method to increase the number of healthy cells, and usually replacing the medium (culture vessel) once or splitting a cell group and culturing the same is called 1 passage. The method of subculturing may be performed without limitation by a method known in the art, but it may be preferably performed by mechanical dissociation or enzymatic dissociation.

In still another specific embodiment of the present invention, in order to confirm the fat transdifferentiation efficiency according to the passage number of muscle stem cells, muscle stem cells at passage 12 were cultured in a medium treated with a combination of substances at various concentration ratios, and then, Oil Red O staining was performed to evaluate the adipocyte transdifferentiation rate. As a result, as shown in FIGS. 5A to 5F, it was confirmed that the adipocyte transdifferentiation rate significantly increased when Combination 1 (oleic acid + rosiglitazone (OR): 200 µM + 5 / 10 / 15 / 20 µM), Combination 2 (oleic acid + lecithin (OL): 200 µM + 7.9 / 15.8 / 23.7 / 31.6 µM) or Combination 4 (oleic acid + rosiglitazone + lecithin (ORL): 200 µM + 5 / 10 / 15 / 20 µM + 7.9 / 15.8 / 23.7 / 31.6 µM) was treated in the standard medium including IBMX and dexamethasone.

FIG. 6 shows a comparison of the adipocyte transdifferentiation rates according to the passage number of muscle stem cells under the same conditions. When Combination 1 (oleic acid + rosiglitazone (OR): 200 µM + 5 / 10 / 15 / 20 µM), Combination 2 (oleic acid + lecithin (OL) 200 µM + 7.9 / 15.8 / 23.7 / 31.6 µM), or Combination 4 (oleic acid + rosiglitazone + lecithin (ORL): 200 µM + 5 / 10 / 15 / 20 µM + 7.9 / 15.8 / 23.7 / 31.6 µM) was treated in a standard medium including insulin, IBMX and dexamethasone, it was confirmed that an excellent adipocyte transdifferentiation rate was shown regardless of the passage number.

FIG. 7 shows a comparison of the adipocyte transdifferentiation rates of muscle stem cells at passages 7 and 12, respectively, according to the concentration ratio of the combination of substances added to the medium in the presence or absence of the chemical mix (insulin, IBMX, and dexamethasone). When Combination 1 (oleic acid + rosiglitazone (OR): 200 µM + 5 / 10 / 15 / 20 µM), Combination 2 (oleic acid + lecithin (OL): 200 µM + 7.9 / 15.8 / 23.7 / 31.6 µM), or Combination 4 (oleic acid + rosiglitazone + lecithin (ORL): 200 µM + 5 / 10 / 15 / 20 µM + 7.9 / 15.8 / 23.7 / 31.6 µM) was treated to the medium including insulin, IBMX and dexamethasone, it was confirmed that overall excellent adipocyte transdifferentiation rates were shown.

As the passage number of cells increases, the transdifferentiation rate generally decreases. However, when the medium composition of the present invention is used, the transdifferentiation rate into adipocytes may be maintained at a high level even when the passage number of muscle stem cells increases.

The cell culture basic medium that can be used in the present invention may be arbitrarily selected from common media used in the relevant field suitable for culturing stem cells. In addition, the culture conditions may also be arbitrarily selected from appropriate conditions used in the relevant field. That is, the medium and culture conditions may be selected according to the type of cells to be cultured. The medium used for culturing is a cell culture minimum medium (CCMM), which can generally include a carbon source, a nitrogen source and trace element components.

The cell culture minimal media that can be used in the present invention include, but are not necessarily limited to, Dulbecco's Modified Eagle's Medium (DMEM), Minimal essential Medium (MEM), Basic medium Eagle (BME), RPMI1640, F-10, F-12, alpha-modified Minimum Essential Media (α-MEM), Glasgow's Minimal essential Medium (GMEM), Iscove's Modified Dulbecco's Medium (IMDM), DMEM/F12 and the like. In the present invention, DMEM/F-12 medium used in cell culture in the art may be used as the basic medium.

Additionally, it is preferable to use in the medium composition of the present invention an antibiotic, an antifungal agent and/or a substance generally used in the relevant industry to prevent the growth of mycoplasma to prevent infection of bacteria, fungi and the like. As the antibiotic, all antibiotics commonly used in cell culture, such as penicillin-streptomycin, may be used, and as the antifungal agent, alporelycin B may be used, and as the mycoplasma inhibitor, a substance generally used, such as gentamicin, ciprofloxacin, azithromycin, may be used, but is not limited thereto. In addition, a commercially available antibiotic-antimycotic (antibiotic 2 antimycotic, AA) (Gibco) may be used.

In addition, the present invention provides a method for transdifferentiating muscle stem cells into adipocytes using the above-described medium composition.

According to a specific embodiment of the present invention, the method for transdifferentiating muscle stem cells into adipocytes may include: (1) a culture medium including two or more selected from the group consisting of oleic acid, thiazolidinedione and lecithin, (2) a culture medium further including one or more additives selected from the group consisting of insulin, IBX and dexamethasone in (1), (3) a culture medium further including serum in (1) or (2), or (4) a medium further including one or more additives selected from the group consisting of insulin, transferrin and selenite in (1).

Since the components included in the culture medium and the effects thereof are the same as those in the above-described medium composition, the description thereof is omitted.

The muscle stem cells used in the method for transdifferentiating muscle stem cells into adipocytes according to the present invention may be undifferentiated muscle stem cells isolated from a non-human animal, and the method for isolating the undifferentiated muscle stem cells may be performed using various methods known in the art, such as using cell growth rate and a sorter.

The method for transdifferentiating muscle stem cells of the present invention into adipocytes may include proliferating the obtained muscle stem cells before culturing the muscle stem cells in the culture medium described above. For example, the method for transdifferentiating muscle stem cells of the present invention into adipocytes may include subculturing the muscle stem cells. As described above, the passage number may be 1 to 25, but is not limited thereto.

According to one specific embodiment of the present invention, the method for transdifferentiating muscle stem cells into adipocytes may include: (a) proliferating muscle stem cells; and (b) culturing the proliferated stem cells in a culture medium defined in (1), (2), (3) or (4) to transdifferentiate muscle stem cells into adipocytes.

According to another specific embodiment of the present invention, the method for transdifferentiating muscle stem cells into adipocytes may include: (a) subculturing muscle stem cells; and (b) culturing the subcultured stem cells in a culture medium defined in (1), (2), (3) or (4) to transdifferentiate muscle stem cells into adipocytes.

The method for transdifferentiating muscle stem cells of the present invention into adipocytes may be, but is not limited to, culturing muscle stem cells in the culture medium described above for 2 to 21 days, and preferably 7 to 14 days.

Additionally, the present invention provides a method for producing cultured meat using the method of transdifferentiating the above-described muscle stem cells into adipocytes, and cultured meat produced thereby.

Specifically, the method for producing cultured meat according to the present invention may include: producing cultured meat by transdifferentiating muscle stem cells into adipocytes by culturing the same in a culture medium defined in (1), (2), (3) or (4) above.

According to a specific embodiment of the present invention, the method for producing cultured meat may include: (a) proliferating muscle stem cells; and (b) culturing the proliferated stem cells in a culture medium defined in (1), (2), (3) or (4) to transdifferentiate the muscle stem cells into adipocytes to produce cultured meat.

According to another specific embodiment of the present invention, the method for transdifferentiating muscle stem cells into adipocytes may include: (a) subculturing muscle stem cells; and (b) culturing the subcultured stem cells in a culture medium defined in (1), (2), (3) or (4) to transdifferentiate muscle stem cells into adipocytes and produce cultured meat.

Here, since the passage number in step (a) and the culture period in step (b) are the same as described above, the description thereof is omitted.

In the present invention, the muscle stem cells used in the method for producing cultured meat are non-human animal muscle stem cells, and may be isolated from chicken, turkey, duck, quail, goose, pigeon, pheasant, ostrich, cow, deer, goat, sheep, horse, llama, camel, pig, rabbit, kangaroo, crocodile, turtle, lobster, salmon, tuna or whale, but are not limited thereto.

In the present invention, in the cultured meat, muscle cells and intramuscular fat may be present simultaneously.

Furthermore, the present invention relates to a food composition including cultured meat produced by the method for producing cultured meat described above.

In the present invention, the food composition may be manufactured as snacks, dumplings, fried foods, stir-fried foods, soy sauce, seasoning, powder mixes, bread, beverages, processed canned foods or processed noodles, but is not limited thereto. The form in which the cultured meat is added to the food may be ground into various particle sizes depending on the purpose of use in the food.

In the present invention, the cultured meat included in the food composition may be additionally treated with a coloring agent. Coloring agents refer to compounds that impart color to foods, and in order to reproduce the red meat color of beef or pork, artificial coloring agents, natural coloring agents, natural extracts (e.g., beet root extract, pomegranate fruit extract, cherry extract, carrot extract, red cabbage extract, red seaweed extract), modified natural extracts, natural juices (e.g., beet root juice, pomegranate juice, cherry juice, carrot juice, red cabbage juice, red seaweed juice), modified natural juices, FD&C (Food Drug Cosmetics) Red No. 3 (erythrosin), FD&C Green No. 3 (fast green FCF), FD&C Red No. 40 (allura red AC), FD&C Yellow No. 5 (tartazine), FD&C Yellow No. 6 (sunset yellow FCF), FD&C Blue No. 1 (brilliant blue FCF), FD&C Blue No. 2 (indigotine), titanium oxide, annatto, anthocyanins, betanins, beta-APE 8 carotenal, beta-carotene, black currant, burnt sugar, canthaxanthin, caramel, carmine/carminic acid, cochineal extract, curcumin, lutein, carotenoids, monascin, paprika, riboflavin, saffron, turmeric, and combinations thereof may be used, but are not particularly limited thereto. Additionally, a coloring agent such as nitrite and ascorbic acid, erythorbic acid or a salt thereof that promotes the coloring of the nitrite may be further added as a coloring aid.

In the present invention, antioxidants, emulsifier salts and the like for stabilizing proteins may be additionally added to the cultured meat included in the food composition to prevent fat oxidation, color change or fat separation. The antioxidants, emulsifier salts and the like may be used without limitation as long as they are widely used in the art.

The food composition including the cultured meat of the present invention may also be manufactured into a synthetic food product. The synthetic food product may further include, in addition to the cultured meat, minerals, vitamins, auxiliary vitamins, essential fatty acids, essential amino acids, enzymes, antioxidants or a combination of two or more thereof.

In addition, synthetic foods including cultured meat may further include flavoring agents, flavor enhancers, sweeteners, coloring agents, colorants, bleaching agents, preservatives, sterilizers, antioxidants, leavening agents, coagulants, or thickening agents.

The form of the synthetic food may be a paste, puree, soup, pie, powder, granule, chip, tablet, capsule, or spread.

The cultured meat produced by the method for producing cultured meat according to the present invention may be specifically processed or manufactured in the form of a meat processed product. The meat processed product may include, but is not limited to, sterilized meat products, ham, pressed ham, mixed pressed ham, sausage, mixed sausage, dried sausage (dried mixed sausage), semi-dried sausage (semi-dried mixed sausage), heated and frozen sausage, bacon, dried stored meat, seasoned meat, ground processed meat, packaged meat, and other meat processed products.

### MODES OF THE INVENTION

### [Example 1]

### Confirmation of fat differentiation efficiency according to type and concentration of additives

### 1-1. Isolation and subculture of chicken muscle stem cells

Chicken muscle stem cells were obtained from cells extracted from the chest and legs of chickens hatched on days 17, 18 and 19 using fertilized eggs cultured in a sterile laboratory.

Specifically, muscle tissues isolated from the breast and leg of chickens were minced into small pieces and treated with collagenase (final concentration 0.1%) in HBSS at 37°C for 45 minutes while continuously stirring. The treated muscle tissues were forcedly separated using a 10-mL pipette, collected by centrifugation (300 g for 5 min), and washed once with PBS. The fragmented tissues were re-treated with 5 mL of trypsin (GIBCO, final concentration 0.1%) at 37°C for 45 minutes while continuously stirring. After treatment, the suspension was diluted with 5 mL of standard medium (DMEM F/12 90%, fetal bovine serum 10%, penicillin (100 IU/mL) and streptomycin (100 µg/mL)) and centrifuged at approximately 300 g for 10 minutes to collect the dissociated cells and tissue fragments. Additional cells trapped in the viscous trypsin supernatant were recovered by diluting the trypsin suspension prior to centrifugation. The final pellet was re-suspended in 5 to 10 mL of standard medium, and the tissue fragments were mechanically dissociated by passing them five times through a 10 mL pipette, then a 5 mL pipette and finally a Pasteur pipette. Cells were collected from the resulting suspension by centrifugation (300 g, 10 min), re-suspended in 2.5 mL of standard medium, and subcultured five times through an 18-gauge needle to separate cell aggregates.

The suspension was then separated into 20% Percoll in 15 mL tubes. The 20% Percoll was added to the tube including 60% Percoll to create a layer and treated with fetal bovine serum for about 2 hours to minimize cell adhesion to the wall. The cell suspension was layered on top of the 20% Percoll and centrifuged at 8°C, 15,000 g for 5 minutes in a centrifuge. After centrifugation, the cells at the 20/60% Percoll interface were collected, diluted with standard medium, and recovered by centrifugation (300 g, 10 min). The cell pellet was re-suspended in 2 mL of standard medium by repeated passage through a Pasteur pipette and additional passage through an 18-gauge needle. The cells were then counted and used for culture.

Cells were placed on collagen-coated tissue culture dishes and pre-incubated with 10% fetal calf serum in DMEM F/12 for 3 hours to promote cell attachment. Cultures were maintained at 39°C in an atmosphere containing 5% CO₂ in air. For large-scale culture, cells were plated at 2Y10⁵ cells per 60 mm dish. For serial passaging of cells, the culture medium was removed, cells were washed with PBS, and then treated with 0.05% trypsin at 37°C for 5 minutes. Subsequently, cells were harvested and centrifuged, and the cell pellet was re-suspended in standard medium. Cells were re-plated at a concentration of 2Y10⁵ per 60 mm dish. Briefly, cells were cultured in 60 mm dishes for 4 to 5 days and then passaged for the next passage.

### 1-2. Preparation of media

The culture medium for transdifferentiation included 900 g/L Dulbecco's Modified Eagle's Medium High glucose (DMEM), which is a serum-free medium, 10% by volume of FBS, 11.2 g/L (500 µM) 3-isobutyl-1-0methylxanthine (IBMX), which is a phosphodiesterase inhibitor, 0.1 g/L (1.72 µM) insulin and 4.0 g/L (0.1 µM) dexamethasone, which is a synthetic glucocorticoid.

Additionally, to determine the adipocyte transdifferentiation rate in the absence of IBMX, insulin and dexamethasone, a basic medium including 900 g/L Dulbecco's Modified Eagle's Medium High glucose (DMEM) and 10 vol% FBS was prepared.

To determine the differentiation efficiency of muscle stem cells into adipocytes according to the type and concentration of additives, the medium was treated with various combinations and concentrations of additives as shown in Table 1 below.

**[Table 1]**

| **Group** | **Basic medium** | **FBS** | **Additives (Presence or absence of insulin, IBMX, dexamethasone addition)** | |
|---|---|---|---|---|
| Control group | DMEM High glucose | 10% FBS | Insulin IBMX dexamethasone | |
| Oleic acid | DMEM High glucose | 10% FBS | 20 / 50 / 100 / 200 µM Oleic acid | Insulin, IBMX, Dexamethasone + 20 / 50 / 100 / 200 µM Oleic acid |
| Rosiglitazon e | DMEM High glucose | 10% FBS | 0.5 / 1 / 5 / 10 µM Rosiglitazone | Insulin, IBMX, dexamethasone + 0.5 / 1 / 5 / 10 µM Rosiglitazone |
| Lecithin | DMEM High glucose | 10% FBS | 1.2 / 2.4 / 6 / 12 µM/ml Lecithin | Insulin, IBMX, Dexamethasone + 1.2 / 2.4 / 6 / 12 µM/ml Lecithin |
| Oleic acid+Rosigli tazone (OR) | DMEM | 10% FBS | 200 µM Oleic acid | Insulin, IBMX, Dexamethasone + 200 µM Oleic acid + 10 µM Rosiglitazone |
| | High glucose | | 10 µM Rosiglitazone | |
| Oleic acid+Lecithi n(OL) | DMEM | 10% FBS | 200 µM Oleic acid | Insulin, IBMX, Dexamethasone + 200 µM Oleic acid + 12µg/ml Lecithin |
| | High glucose | | 12µg/ml Lecithin | |
| Rosiglitazon e+Lecithin( RL) | DMEM | 10% FBS | 10 µM Rosiglitazone | Insulin, IBMX, Dexamethasone + 10 µM Rosiglitazone + 12µg/ml Lecithin |
| | High glucose | | 12µg/ml Lecithin | |
| Oleic acid+Rosigli tazone+ Lecithin(OR L) | DMEM | 10% FBS | 200 µM Oleic acid | Insulin, IBMX, Dexamethasone + 200 µM Oleic acid + 10 µM Rosiglitazone + 12µg/ml Lecithin |
| | High glucose | | 10 µM Rosiglitazone | |
| | | | 12µg/ml Lecithin | |

### 1-3. Culturing of muscle stem cells

Chicken muscle stem cells prepared in Example 1-1 were cultured in DMEM high glucose medium including 10% FBS at 39°C for 3 days, and then seeded at 2.1 × 10⁴ cells/well in a 12-well plate. The day after seeding, they were cultured in culture medium according to the conditions of each group in Table 1 at 39°C for 14 days.

### 1-4. Oil Red O Staining

After removing the medium from the adipocytes transdifferentiated from chicken muscle stem cells, DPBS was added to wash the cells, and it was mixed well and removed. Next, 4% formalin solution was added at room temperature, and left for at least 1 hour. Next, after removing the formalin, the cells were washed three times with distilled water, isopropanol at a concentration of 60% by volume was added, it was left for 5 minutes, isopropanol was removed, and it was dried completely. Oil Red O working solution was added, and it was left for 30 minutes at room temperature. Oil Red O solution was removed, and triple-distilled water was immediately added. After observing under a microscope, it was confirmed that the cells were stained, and then, photographs were taken. After discarding the water, the flask was dried, 100% isopropanol was added, and it was shaken slowly for 10 minutes to completely dissolve the Oil Red O. Absorbance was measured at 490 nm using a microplate reader, and absorbance was also measured at 630 nm to correct the absorbance at 490 nm.

As a result, as confirmed in FIGS. 1, 2A and 2B, when IBMX, insulin and dexamethasone were included, the adipocyte transdifferentiation efficiency increased as the concentration of oleic acid, rosiglitazone and/or lecithin increased, and the best adipocyte transdifferentiation rate was exhibited when IBMX, insulin, dexamethasone, oleic acid, rosiglitazone and lecithin were all included. In addition, as confirmed in FIGS. 1, 3A and 3B, when IBMX, insulin and dexamethasone were not included, the adipocyte transdifferentiation efficiency decreased compared to when IBMX, insulin and dexamethasone were included, but when two or more of oleic acid, rosiglitazone and lecithin were added, the adipocyte transdifferentiation efficiency tended to increase.

### [Example 2]

### Confirmation of fat differentiation efficiency according to additive combination concentration

### 2-1. Isolation and subculture of chicken muscle stem cells

Chicken muscle stem cells at passage 7 using the same method as in Example 1-1 were prepared.

### 2-2. Preparation of media and culturing of muscle stem cells

In the same manner as in Example 1-2, the medium was prepared, and to confirm the efficiency of transdifferentiation of muscle stem cells into adipocytes according to the combined concentrations of additives, oleic acid + rosiglitazone (Combination 1), oleic acid + lecithin (Combination 2), rosiglitazone + lecithin (Combination 3) and oleic acid + rosiglitazone + lecithin (Combination 4) were treated in various concentration combinations as shown in Table 2 below in DMEM medium including IBMX, insulin and dexamethasone (insulin 1.72 µM, IBMX 500 µM and dexamethasone 0.1 µM) and DMEM medium not including IBMX, insulin, and dexamethasone. As a control group, DMEM medium including FBS was treated with insulin, IBMX and dexamethasone and used.

**[Table 2]**

| **Group** | **Basic medium** | **FBS** | **Additives (Presence or absence of insulin, IBMX, dexamethasone addition)** | |
|---|---|---|---|---|
| Control group | DMEM High glucose | 10% FBS | Insulin, IBMX, Dexamethasone | |
| Combination 1(OR): Oleic acid+ Rosiglitazone (200 µM + 5 / 10 / 15 / 20 µM) | DMEM High glucose | 10% FBS | Combination 1 | Insulin, IBMX, Dexamethasone + Combination 1 |
| Combination 2(OL): Oleic acid+Lecithin(200 µM + 7.9 / 15.8 / 23.7 / 31.6 µM) | DMEM High glucose | 10% FBS | Combination 2 | Insulin, IBMX, Dexamethasone + Combination 2 |
| Combination 3(RL): Rosiglitazone + Lecithin (5 / 10 / 15 / 20 µM + 7.9 / 15.8 / 23.7 / 31.6 µM) | DMEM High glucose | 10% FBS | Combination 3 | Insulin, IBMX, Dexamethasone + Combination 3 |
| Combination 4(ORL): Oleic acid+ Rosiglitazone + Lecithin (200 µM + 5 / 10 / 15 / 20 µM + 7.9 / 15.8 / 23.7 / 31.6 µM) | DMEM High glucose | 10% FBS | Combination 4 | Insulin, IBMX, Dexamethasone + Combination 4 |

Chicken muscle stem cells at passage 7 were cultured in DMEM high glucose medium including 10% FBS at 39°C for 3 days, and then seeded at 2.1 × 10⁴ cells/well in 12-well plates. The day after seeding, the cells were cultured in culture medium according to the conditions of each group in Table 2 at 39°C for 14 days, and the adipocyte differentiation rate was confirmed by Oil Red O staining as in Example 1-4.

FIG. 4A shows the results of muscle stem cells cultured in a medium treated with insulin, IBMX and dexamethasone, confirming that transdifferentiation into adipocytes did not proceed.

FIG. 4B shows the results for Combination 1, which confirmed that the adipocyte transdifferentiation rate was significantly increased regardless of the inclusion of insulin, IBMX and dexamethasone, and in particular, when Combination 1 was included in DMEM high glucose medium including insulin, IBMX and dexamethasone, excellent adipocyte transdifferentiation rates were shown in all concentration combinations.

FIG. 4C shows the results for Combination 2, which confirmed that the adipocyte transdifferentiation rate was significantly increased regardless of the inclusion of insulin, IBMX and dexamethasone, and in particular, when Combination 2 was included in DMEM high glucose medium including insulin, IBMX and dexamethasone, excellent adipocyte transdifferentiation rates were shown in all concentration combinations.

FIG. 4D shows the results for Combination 3, which showed that the adipocyte transdifferentiation rate tended to increase when Combination 3 was included in DMEM high glucose medium including insulin, IBMX and dexamethasone, but the effect was minimal compared to the combinations of other groups.

As a result for Combination 4 in FIG. 4E, it was confirmed that the adipocyte transdifferentiation rate was significantly increased regardless of the inclusion of insulin, IBMX and dexamethasone, and especially when Combination 4 was included in the DMEM high glucose medium including insulin, IBMX and dexamethasone, excellent adipocyte transdifferentiation rates were shown in all concentration combinations.

FIG. 4F quantitatively shows the results of Oil Red O staining of each group above, and it can be confirmed that the adipocyte transdifferentiation rate significantly increased when Combination 1, Combination 2 or Combination 4 was included in the DMEM high glucose medium including insulin, IBMX and dexamethasone.

### [Example 3]

### Confirmation of fat differentiation efficiency according to the passage number of cells

Chicken muscle stem cells at passage 12 in the same manner as in Example 1-1 were prepared, and the adipocyte transdifferentiation rate was confirmed in the same manner as in Example 2.

FIG. 5A shows the results of muscle stem cells cultured in a medium treated with insulin, IBMX and dexamethasone, confirming that transdifferentiation into adipocytes did not proceed.

FIG. 5B shows the results for Combination 1, which confirmed that the adipocyte transdifferentiation rate was significantly increased regardless of the inclusion of insulin, IBMX and dexamethasone, and in particular, when Combination 1 was included in DMEM high glucose medium including insulin, IBMX and dexamethasone, excellent adipocyte transdifferentiation rates were shown in all concentration combinations.

FIG. 5C shows the results for Combination 2, which confirmed that the adipocyte transdifferentiation rate was significantly increased regardless of the inclusion of insulin, IBMX and dexamethasone, and in particular, when Combination 2 was included in DMEM high glucose medium including insulin, IBMX and dexamethasone, excellent adipocyte transdifferentiation rates were shown in all concentration combinations.

FIG. 5D shows the results for Combination 3, which showed that the adipocyte transdifferentiation rate tended to increase when Combination 3 was included in DMEM high glucose medium including insulin, IBMX and dexamethasone, but the effect was minimal compared to the combinations of other groups.

As a result for Combination 4 in FIG. 5E, it was confirmed that the adipocyte transdifferentiation rate was significantly increased regardless of the inclusion of insulin, IBMX and dexamethasone, and especially when Combination 4 was included in the DMEM high glucose medium including insulin, IBMX and dexamethasone, excellent adipocyte transdifferentiation rates were shown in all concentration combinations.

FIG. 5F quantitatively shows the results of oil red O staining of each group above, and it can be confirmed that the adipocyte transdifferentiation rate significantly increased when Combination 1, Combination 2 or Combination 4 was included in the DMEM high glucose medium including insulin, IBMX and dexamethasone.

FIG. 6 shows the results of Examples 2 and 3 compared according to the passage number, and it was confirmed that when Combination 1, Combination 2 or Combination 4 was included in the DMEM high glucose medium including insulin, IBMX and dexamethasone, excellent adipocyte transdifferentiation rates were shown regardless of the passage number.

FIG. 7 compares the results of Examples 2 and 3 depending on the presence or absence of chemical mix (insulin, IBMX and dexamethasone). Similarly, it was confirmed that when Combination 1, Combination 2 or Combination 4 was included in the DMEM high glucose medium including insulin, IBMX and dexamethasone, overall superior adipocyte transdifferentiation rates were shown.

### [Example 4]

### Confirmation of effects of adipocyte transdifferentiation according to presence or absence of serum in medium

Instead of 10 vol% of FBS, DMEM/F12 medium including 1% insulin-transferrin-selenite (ITS) was treated with 200 µM of oleic acid, 10 µM of rosiglitazone, 12 µg/mL of lecithin, 0.5 mM of IBMX, 0.1 µM of dexamethasone and 10 µg/mL of insulin, and then, muscle stem cells were cultured in the same manner as in Examples 1-3, and the efficiency of adipocyte transdifferentiation was confirmed by Oil Red O staining.

As a result, as confirmed in FIG. 8, even under conditions without serum, the adipocyte transdifferentiation rate was 96% compared to the condition with serum, indicating that the combination of insulin, IBMX, dexamethasone, oleic acid, rosiglitazone and lecithin effectively induces transdifferentiation into adipocytes.

While the specific parts of the present invention have been described in detail above, it will be apparent to those skilled in the art that such specific descriptions are merely preferred embodiments and that the scope of the present invention is not limited thereby. Accordingly, the actual scope of the present invention will be defined by the appended claims and their equivalents.

## Claims

1. A culture medium composition for transdifferentiating muscle stem cells into adipocytes, comprising two or more selected from the group consisting of oleic acid, thiazolidinedione and lecithin.

2. The culture medium composition of claim 1, wherein the thiazolidinedione is selected from the group consisting of pioglitazone, rosiglitazone, lobeglitazone, troglitazone, ciglitazone, darglitazone, englitazone, netoglitazone and rivoglitazone.

3. The culture medium composition of claim 1, wherein the oleic acid is comprised at a concentration of 100 to 250 µM.

4. The culture medium composition of claim 1, wherein the thiazolidinedione is comprised at a concentration of 5 to 20 µM.

5. The culture medium composition of claim 1, wherein the lecithin is comprised at a concentration of 5 to 35 µM.

6. The culture medium composition of claim 1, wherein when the culture medium composition comprises oleic acid and thiazolidinedione, the oleic acid and thiazolidinedione are comprised in a concentration ratio of 40 : 0.5 to 6.

7. The culture medium composition of claim 1, wherein when the culture medium composition comprises oleic acid and lecithin, the oleic acid and lecithin are comprised in a concentration ratio of 40 : 1 to 7.

8. The culture medium composition of claim 1, wherein when the culture medium composition comprises oleic acid, thiazolidinedione and lecithin, the oleic acid, thiazolidinedione and lecithin are comprised in a concentration ratio of 40 : 0.5 to 6 : 1 to 7.

9. The culture medium composition of claim 1, further comprising one or more selected from the group consisting of insulin, 3-isobutyl-1-methylxanthine (IBMX) and dexamethasone.

10. The culture medium composition of claim 1, wherein the culture medium composition does not comprise serum or further comprises serum.

11. The culture medium composition of claim 10, wherein when the culture medium composition does not comprise serum, the culture medium composition further comprises one or more selected from the group consisting of insulin, transferrin and selenite.

12. The culture medium composition of claim 1, wherein the muscle stem cells are passaged 1 to 25 times.

13. A method for transdifferentiating muscle stem cells into adipocytes using the culture medium composition according to any one of claims 1 to 12.

14. A method for producing cultured meat, using the method of claim 13.

15. A cultured meat, produced by the method of claim 14.

16. The cultured meat of claim 15, wherein in the cultured meat, muscle cells and intramuscular fat are present simultaneously.

17. A food composition, comprising the cultured meat of claim 15 or claim 16.
